# EUROPEAN PATENT APPLICATION

(11) **EP 2 144 062 A1**
(43) Date of publication of application: **13.01.2010**
(21) Application number: 08156271.2
(22) Date of filing: 15.05.2008
(51) Int. Cl.: G01N 33/50

(54) **Method of monitoring live cells**

(71) Applicant: Biocompatibles UK Limited, Farnham Surrey GU9 8QL (GB)
(72) Inventor: Battaglia, Giuseppe, Sheffield, S1 7HQ (GB)
(74) Representative: Jones, Helen M.M.

(57) **Abstract**

The present invention relates to a method of monitoring live cells, comprising the steps of:
(i) contacting the cells with self-assembled constructs associated with an imaging agent;
(ii) incubating the cells;
(iii) imaging the live cells;

wherein in step (i), at least some of the constructs are taken up into the cells;
and wherein the constructs comprise an amphiphilic block copolymer having a hydrophilic and hydrophobic block.

## Description

### Field of the Invention

The present invention relates to a method of monitoring live cells in which the cells are contacted with nanovesicles associated with an imaging agent. The nanovesicles comprise an amphiphilic block copolymer.

### Background of the Invention

Intracellular or cytosolic delivery of active agents is one the most challenging delivery mechanisms to achieve, and involves the active transport of molecules or macromolecules across the cellular membrane ideally without compromising their integrity. To date, intracellular structures and organelles have been accessed by controlled membrane permeabilisation based on several techniques such as organic solvent exposure, surfactant exposure, ultrasound, and electroporation. These techniques, however, often result in cell toxicity.

Liposomes have been widely studied for drug delivery within cells. However, the bilayer membranes of liposomes are relatively thin and fragile, which leads to facile vesicle fusion and inadequate barrier properties. Recently, micellar self-assembly techniques have been used to prepare block copolymer vesicles. Our previous patent application, published as WO 03/074026 describes a drug carrier in the form of micelles formed from an amphiphilic block copolymer having a hydrophilic block comprising pendant zwitterionic groups, and a hydrophobic block.

Polymeric nanovesicles, also known as polymersomes (Discher et al; Science, 1999, 284, 1143-1146) result from the spontaneous assembly of amphiphilic copolymer in water.

Du, J et al in "pH-Sensitive Vesicles Based on a Biocompatible Zwitterionic Diblock Copolymer" in J. Am. Chem. Soc. 2005, 127, 17982-17983 describe diblock copolymers based on a highly biocompatible monomer, 2-(methacryloyloxy)ethyl phosphorylcholine (MPC), and a second monomer, 2-(diisopropylamino)ethyl methacrylate (DPA) and its use to form vesicles having a bilayer structure with a sandwich of DPA derived polymer between two MPC derived polymer layers. Doxorubicin is encapsulated in the vesicles and release profiles are obtained.

In a poster presented at the International Symposium on Polymer Therapeutics, at the Freie Universität Berlin, February 19-21, 2007, entitled "Polymer Vesicles for Efficient Intracellular Delivery", polymer vesicles based on synthetic amphiphilic block copolymers were described. The diblock copolymers were pH sensitive and formed vesicles with a bilayer structure when hydrated with aqueous liquid at neutral pH. When the pH of the medium was reduced to endocytic pH the polymers dissolved completely as unimers. Fluorescently-labelled vesicles were delivered into cells. GFP (Green fluoresescent protein)-encoding plasmid DNA was also delivered into cells using the nanovesicles and was expressed to give GFP in the cell cytosol. In the abstract accompanying the poster, uptake of rhodamine by non-pH sensitive polyethylene oxide-polybutylene oxide (PEO-PBO) vesicles was compared with uptake by pH sensitive PMPC-PDPA vesicles.

US6,835,394 describes biocompatible vesicles formed from one or more "super-amphiphilic" molecules, which may be block copolymers. The copolymers may include polyethylene oxide (PEO), polyethyl ethylene (PEE) and polybutadiene (PB), amongst other polymers. The vesicles are used to encapsulate compounds such as drugs, dyes, vitamins and genes. An example at a preferred block copolymer from this Patent is (EO₄₀-EE₃₇). US6,835,394 does not describe the monitoring of cells over time.

Fluorescence staining of cells is one the most used and important biological techniques for gathering information about the status, the morphology, and the nature of a particular cell. Fluorescence staining is normally performed by incubating cells with bioactive molecules that have been tagged to a fluorophore. These bioactive molecules are either capable of targeting a specific part of a cell (e.g. DNA binding complex or fluorescent antibodies) or react in the presence of specific enzymes or other cytosolic molecules (e.g. CellTracker™ or Calcium indicator). There is a strong desire to target fluorophores to structures inside cells and consequently, the efficient and effective delivery of fluorophores into the cells, is fundamental. To date, most of the fluorescence staining techniques are based on the fixation of cells and controlled permeabilisation of their membranes. However, as research in cell biology, neuroscience and immunology has progressed from purely structural characterisation of cells towards understanding dynamic processes, such as signaling, excitability, growth and metabolic transport, fixed cell fluorescence detection techniques are no longer sufficient.

Techniques have been developed to monitor live cells. An example of a conventional probe used to visualise live cells is the family of CellTracker™ probes from Molecular Probes™. These are fluorescent chloromethyl derivatives, which are processed into membrane impermeable compounds that are retained inside the cells up to 72 hours after loading, and can be passed to daughter cells.

Unfortunately, the CellTracker™ probes do have some disadvantages. The probes are used in DMSO solution, which may affect the viability of sensitive cell lines. In addition, it is not known whether the dyes adversely affect intracellular components.

In view of the prior art, there is therefore a need to provide improved tracking products for monitoring live cells.

### Summary of the Invention

In accordance with this invention there is provided a method of monitoring live cells, comprising the steps of:
(i) contacting the cells with self-assembled constructs associated with an imaging agent;
(ii) incubating the cells;
(iii) imaging the live cells;
wherein in step (i), at least some of the constructs are taken up into the cells;
and wherein the constructs comprise an amphiphilic block copolymer having a hydrophilic and hydrophobic block.

Once the self-assembled constructs are taken up into cells, they typically dissociate and release imaging agent within the cell. Dissociation may be promoted by a variety of mechanisms, but is typically promoted by the pH sensitivity of the block copolymer. Preferably, one of the blocks, and typically the hydrophobic block of the copolymer has pendant groups having a pKₐ in the range 3.0 to 6.9, and preferably 4.0 to 6.9. The mechanism of cell internalisation (endocytosis) of the self-assembled constructs involves engulfment within phospholipid membranes produced by endocytic organelles such as trafficking vesicles, phagosomes, or pinosomes, depending on the precise endocytic pathway. The endocytic organelle detaches from the cell membrane and takes the constructs inside the cell for further processing. Regardless of the endocytic pathway, the internalised constructs experience a reduction in local pH from pH 7.4 to pH 5-6 once inside the organelle. This pH drop is sufficient to trigger disintegration of the constructs and release of the imaging agent. As this transition is confined within a semi-permeable organelle membrane, the sudden increase in particle number corresponds to a large increase in osmotic pressure. This causes lysis of the lipid membrane, releasing the imaging agent into the cell cytosol.

It is preferred that the self-assembled constructs are vesicles rather than micelles, since vesicles are typically formed from a greater number of amphiphilic copolymer units than micelles and have water molecules encapsulated within their core. Although the pH drop inside the organelle is sufficient to cause micelles to dissociate, this does not cause a large enough increase in particles to cause lysis of the membrane of the organelle. Thus an encapsulated compound in micelles will not generally be released into the cell cytosol.

An advantage of the copolymers used in the present invention is that they do not undergo any cytotoxic interactions with cells.

The delivery of imaging agents using the constructs of the present invention is more efficient than the delivery systems of the prior art, thereby allowing a larger amount of imaging agent to be delivered and improved images from the cells to be obtained.

None of the above-mentioned prior art disclosures describe use of self-assembled constructs comprising block copolymers to monitor live cells over a period of time.

In accordance with a second aspect of the invention there is provided a composition comprising self-assembled constructs, and associated with the constructs, an imaging agent, wherein the constructs comprise an amphiphilic block copolymer having a hydrophilic and hydrophobic block;
and wherein the imaging agent is a biomolecule, or derived from a biomolecule, and is capable of targeting a specific cellular structure.

It is believed that this is the first disclosure of a composition comprising vesicles having an imaging agent which specifically targets a cellular structure. Such specificity allows the internal trafficking processes of cells to be closely monitored.

### Detailed Description of the Invention

In the detailed description which follows, it is to be assumed that the specification is referring to both the first and the second aspects of the invention, unless stated otherwise.

By "self-assembled construct", we mean any structure formed by the amphiphilic block copolymer. The composition used in this invention is generally an aqueous composition and the constructs are in aqueous solution. The aqueous composition generally has a pH which is in the range 7.0 to 7.6, more typically 7.2 to 7.4. The copolymer typically forms membrane-enclosed structures, such as vesicles (otherwise known as polymersomes) or lamellarsomes. Micelles may alternatively be formed. The constructs may be substantially spherical or alternatively tubular or cochleate in shape.

Preferably, the constructs are vesicles. Vesicles have a bilayered membrane formed from copolymer and an aqueous core. A typical diameter of a substantially spherical vesicle is in the range 50-5000nm. More typically, the diameter is in the range 50-1000nm. Vesicles having a diameter in this range are normally termed "nanovesicles". The nanovesicles are preferably substantially spherical in shape. Typically, the nanovesicles have a number average diameter of less than 300nm, preferably less than 250nm, most preferably less than 200nm or 150nm. The thickness of the bilayer is generally between 2 to 50nm, more typically between 5 and 20nm. These dimensions can be measured by Transmission Electron Microscopy (T.E.M) and Small Angle X-ray Scattering (SAXS) (Battaglia et al; JACS 127, 8757 (2005).

In aqueous solution, normally an equilibrium exists between different types of structures, for instance between vesicles and micelles. It is preferred that at least 80%, more preferably at least 90% by weight of the structures in solution are present as vesicles. This can be achieved using the methods outlined in this specification below.

As detailed above, the hydrophobic or the hydrophilic block, preferably the hydrophobic block preferably comprises pendant groups which have a pKₐ in the range 3.0 to 6.9. By pKₐ, is meant the pH where half of the pendant groups are ionised. pKₐ can be determined by a variety of methods including pH titration followed by potentiometric titration, UV spectroscopy and Dynamic Light Scattering (DLS). An appropriate method should be selected to measure the pKₐ according to the copolymer which is being analysed and its solubility in the test media.

DLS is the particularly preferred method for measuring pKₐ. As indicated in the paper by Du et a/; J. Am. Chem. Soc 2005, 127, 17982-17983, the DLS signal from PMPC₂₅-*b*-PDPA₁₂₀ copolymer in water varies with pH. At a certain pH the signal rapidly increases as the copolymer undergoes a transition from being molecularly dissociated to associated. The pKₐ is taken as the pH of the mid-point of this rapid increase. These experiments are described further in Giacomelli et al, Biomacromolecules 2006, 7, 817-828. In this reference, the experiments are performed on micelles of PMPC-b-PDPA block copolymer, but the techniques may also be used when the phase transition involves vesicle formation.

Preferably, the pKₐ of the pendant groups is in the range 4.0 to 6.9, more preferably 5.5 to 6.9. The self-assembled constructs are correspondingly capable of dissociating in such pH ranges.

In the specification, the pKₐ of a group in a polymer is determined on the basis of a polymer system (and not assumed to be the same as the pKₐ's of similar moieties in non-polymeric systems).

It is preferred that the hydrophobic block comprise pendant cationisable moieties as pendant groups. Cationisable moieties are, for instance, primary, secondary or tertiary amines, capable of being protonated at pH's below a value in the range 3 to 6.9. Alternatively the group may be a phosphine.

In one embodiment of the invention, the hydrophobic block has a degree of polymerisation of at least 70. Preferably, the degree of polymerisation of the hydrophobic block is no more than 250, even more preferably, no more than 200. Typically, the degree of polymerisation of the hydrophilic block is at least 15, more preferably at least 20. It is preferred that, the ratio of the degree of polymerisation of the hydrophilic to hydrophobic block is in the range 1:2.5 to 1:8. All of these limitations promote vesicle, rather than micelle formation.

In the invention, although the hydrophilic block may be based on condensation polymers, such as polyesters, polyamides, polyanhydrides, polyurethanes, polyethers (including polyalkylene glycols), polyimines, polypeptides, polyureas, polyacetals or polysaccharides, preferably the hydrophilic block is based on a radical polymerised addition polymer of ethylenically unsaturated monomers. Generally the monomers from which the block is formed themselves have zwitterionic pendant groups which remain unchanged in the polymerisation process. It may alternatively be possible to derivatise a functional pendant group of a monomer to render it zwitterionic after polymerisation.

Preferably, the hydrophilic block is a polyalkylene oxide, most preferably polyethylene oxide. In a different embodiment of the invention, suitable ethylenically unsaturated zwitterionic monomers have the general formula

Y B X I

In which Y is an ethylenically unsaturated group selected from H₂C=CR-CO-A-, H₂C=CR-C₆H₄-A¹-, H₂C=CR-CH₂A², R²O-CO-CR=CR-CO-O, RCH=CH-CO-O-, RCH=C(COOR²)CH₂-CO-O, 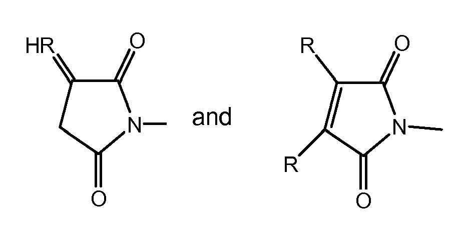
A is -O- or NR¹;
A¹ is selected from a bond, (CH₂)_{I}A² and (CH₂)_{I} SI₃⁻ in which I is 1 to 12;
A² is selected from a bond, -O-, O-CO-, CO-O, CO-NR¹-, -NR¹-CO, O-CO-NR¹-, NR¹-CO-O-;
R is hydrogen or C₁₋₄ alkyl;
R¹ is hydrogen, C₁₋₄₋alkyl or BX;
R² is hydrogen or C₁₋₄ alkyl;
B is a bond, or a straight branched alkanediyl, alkylene oxaalkylene, or alkylene (oligooxalkylene) group, optionally containing one or more fluorine substituents;
X is a zwitterionic group.

Preferably X is an ammonium, phosphonium, or sulphonium phosphate or phosphonate ester zwitterionic group, more preferably a group of the general formula II 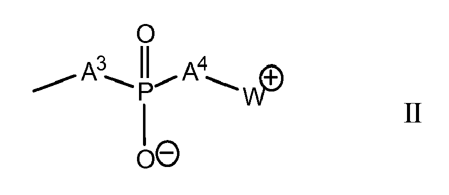 in which the moieties A³ and A⁴, which are the same or different, are -O-, -S-, -NH- or a valence bond, preferably -O-, and W⁺ is a group comprising an ammonium, phosphonium or sulphonium cationic group and a group linking the anionic and cationic moieties which is preferably a C₁₋₁₂-alkanediyl group,
preferably in which W⁺ is a group of formula
-W¹-N⁺R³₃, -W¹-P⁺R⁴₃, -W¹-S⁺R⁴₂ or -W¹-Het in which:
W¹ is alkanediyl of 1 or more, preferably 2-6 carbon atoms optionally containing one or more ethylenically unsaturated double or triple bonds, disubstituted-aryl (arylene), alkylene arylene, arylene alkylene, or alkylene aryl alkylene, cycloalkanediyl, alkylene cycloalkyl, cycloalkyl alkylene or alkylene cycloalkyl alkylene, which group W¹ optionally contains one or more fluorine substituents and/or one or more functional groups; and
   either the groups R³ are the same or different and each is hydrogen or alkyl of 1 to 4 carbon atoms, preferably methyl, or aryl, such as phenyl, or two of the groups R³ together with the nitrogen atom to which they are attached form an aliphatic heterocyclic ring containing from 5 to 7 atoms, or the three groups R³ together with the nitrogen atom to which they are attached as heteroaromatic ring having 5 to 7 atoms, either of which rings may be fused with another saturated or unsaturated ring to form a fused ring structure containing from 5 to 7 atoms in each ring, and optionally one or more of the groups R³ is substituted by a hydrophilic functional group, and
   the groups R⁴ are the same or different and each is R³ or a group OR³,
   where R³ is as defined above; or
   Het is an aromatic nitrogen-, phosphorus- or sulphur-, preferably nitrogen-, containing ring, for example pyridine.
   Monomers in which X is of the general formula in which W⁺ is W¹N⁺R³₃ may be made as described in our earlier specification WO-A-9301221. Phosphonium and sulphonium analogues are described in WO-A-9520407 and WO-A-9416749.

Generally a group of the formula II has the preferred general formula III 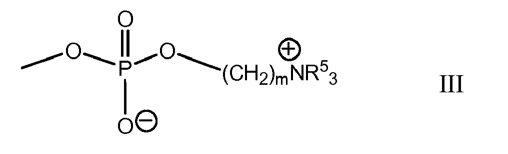 where the groups R⁵ are the same or different and each is hydrogen or C₁₋₄ alkyl, and m is from 1 to 4, in which preferably the groups R⁵ are the same preferably methyl.

In phosphobetaine based groups, X may have the general formula IV 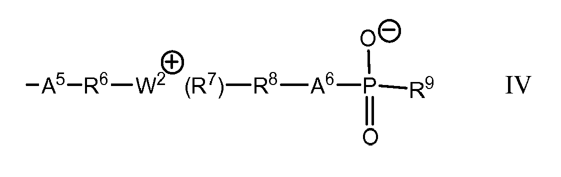 in which A⁵ is a valence bond, -O-, -S- or -NH-, preferably -O-;
R⁶ is a valence bond (together with A⁵) or alkanediyl, -C(O)alkylene- or - C(O)NH alkylene preferably alkanediyl, and preferably containing from 1 to 6 carbon atoms in the alkanediyl chain;
W² is S, PR⁷ or NR⁷;
   the or each group R⁷ is hydrogen or alkyl of 1 to 4 carbon atoms or the two groups R⁷ together with the heteroatom to which they are attached form a heterocyclic ring of 5 to 7 atoms;
R⁸ is alkanediyl of 1 to 20, preferably 1 to 10, more preferably 1 to 6 carbon atoms;
A⁶ is a bond, NH, S or O, preferably O; and
R⁹ is a hydroxyl, C₁₋₁₂ alkyl, C₁₋₁₂ alkoxy, C₇₋₁₈ aralkyl, C₇₋₁₈ -aralkoxy, C₆₋₁₈ aryl or C₆₋₁₈ aryloxy group.

Monomers comprising a group of the general formula IV may be made by methods as described in JP-B-03-031718, in which an amino substituted monomer is reacted with a phospholane.

In compounds comprising a group of the general formula IV, it is preferred that
A⁵ is a bond;
R⁶ is a C₂₋₆ alkanediyl;
W² is NR⁷:
   each R⁷ is C₁₋₄ alkyl;
R⁸ is C₂₋₆ alkanediyl;
A⁶ is O; and
R⁹ is C₁₋₄ alkoxy.

Alternatively X may be a zwitterion in which the anion comprises a sulphate, sulphonate or carboxylate group.

One example of such a group is a sulphobetaine group, of the general formula V 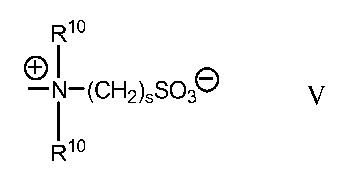 where the groups R¹⁰ are the same or different and each is hydrogen or C₁₋₄ alkyl and s is from 2 to 4.

Preferably the groups R¹⁰ are the same. It is also preferable that at least one of the groups R¹⁰ is methyl, and more preferable that the groups R³⁶ are both methyl.

Preferably s is 2 or 3, more preferably 3.

Another example of a zwitterionic group having a carboxylate group is an amino acid moiety in which the alpha carbon atom (to which an amine group and the carboxylic acid group are attached) is joined through a linker group to the backbone of the biocompatible polymer. Such groups may be represented by the general formula VI 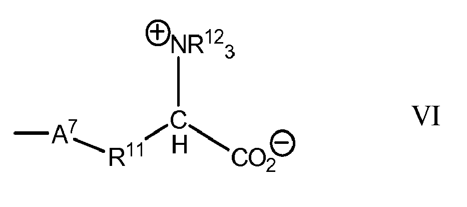 in which A⁷ is a valence bond, -O-, -S- or -NH-, preferably -O-,
R¹¹ is a valence bond (optionally together with A⁷) or alkanediyl, - C(O)alkylene- or -C(O)NHalkylene, preferably alkanediyl and preferably containing from 1 to 6 carbon atoms; and
the groups R¹² are the same or different and each is hydrogen or alkyl of 1 to 4 carbon atoms, preferably methyl, or two or three of the groups R¹², together with the nitrogen to which they are attached, form a heterocyclic ring of from 5 to 7 atoms, or the three group R¹² together with the nitrogen atom to which they are attached form a fused ring heterocyclic structure containing from 5 to 7 atoms in each ring.

Another example of a zwitterion having a carboxylate group is a carboxy betaine -N⁺(R¹³)₂(CH₂)ᵣCOO- in which the R¹³ groups are the same or different and each is hydrogen or R₁₋₄ alkyl and r is 2 to 6, preferably 2 or 3.

In the zwitterionic monomer of the general formula I it is preferred that the ethylenic unsaturated group Y is H₂C=CR-CO-A-. Such acrylic moieties are preferably methacrylic, that is in which R is methyl, or acrylic, in which R is hydrogen. Whilst the compounds may be (meth)acrylamido compounds (in which A is NR¹), in which case R¹ is preferably hydrogen, or less preferably, methyl, most preferably the compounds are esters, that is in which A is O.

In monomers of the general formula I, especially where Y is the preferred (alk)acrylic group, B is most preferably an alkanediyl group. Whilst some of the hydrogen atoms of such group may be substituted by fluorine atoms, preferably B is an unsubstituted alkanediyl group, most preferably a straight chain group having 2 to 6 carbon atoms.

A particularly preferred zwitterionic monomer is 2-methacryloyloxyethylphosphorylcholine (MPC). Mixtures of zwitterionic monomers each having the above general formula may be used.

The hydrophobic block may be formed of condensation polymers, such as polyethers (including polyalkylene glycols), polyesters, polyamides, polyanhydrides, polyurethanes, polyimines, polypeptides, polyureas, polyacetals, or polysiloxanes. One example of a suitable hydrophobic block is polyalkylene oxide, usually polypropylene oxide, that is the same type of block as has been used in the well-studied Pluronic/Poloxamer based systems. One type of highly hydrophobic block is poly(dimethylsiloxane). In one preferred embodiment the type of polymer forming the hydrophobic block is the same as that forming the hydrophilic block. Preferably the polymer is formed by radical polymerisation of ethylenically unsaturated monomers.

Suitable monomers from which the hydrophobic block may be formed have the general formula VII

Y¹B¹Q VII

in which Y¹ is selected from H₂C=CR¹⁴-CO-A⁸-, H₂C=CR¹⁴-C₆H₄-A⁹-, H₂C=CR¹⁴-CH₂A¹⁰, R¹⁶O-CO-CR¹⁴=CR¹⁴-CO-O, R¹⁴CH=CH-CO-O-, R¹⁴CH=C(COOR¹⁶)CH₂-CO-O, 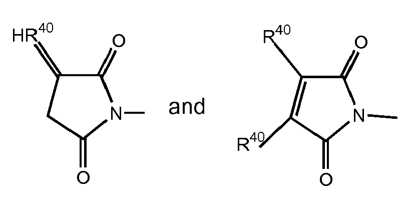
A⁸ is -O- or NR¹⁵;
A⁹ is selected from a bond, (CH₂)_{q}A¹⁰ and (CH₂)_{q} SO₃⁻ in which q is 1 to 12;
A¹⁰ is selected from a bond, -O-, O-CO-, CO-O-, CO-NR¹⁵-, -NR¹⁵-CO-, O-CO-NR¹⁵-, NR¹⁵-CO-O-;
R¹⁴ is hydrogen or C₁₋₄ alkyl;
R¹⁵ is hydrogen, C₁₋₄-alkyl or B¹Q;
R¹⁶ is hydrogen or C₁₋₄ alkyl;
B¹ is a bond, or a straight branched alkanediyl, alkylene oxaalkylene, or alkylene (oligooxalkylene) group, optionally containing one or more fluorine substituents; and
Q is a cationic or cationisable group of the formula -NR¹⁷ₚ, -PR¹⁷ₚ and SR¹⁷ᵣ, in which p is 2 or 3, r is 1 or 2, the groups R⁴³ are the same or different and each is selected from the group consisting of hydrogen, C₁₋₂₄ alkyl and aryl, or two of the groups R¹⁷ together with the heteroatom to which they are attached from a 5 to 7 membered heterocyclic ring or three R¹⁷ groups together with the heteroatom to which they are attached form a 5 to 7 membered heteroaromatic ring, either of which rings may be fused to another 5 to 7 membered saturated or unsaturated ring, and any of the R⁴³ groups may be substituted by amino or hydroxyl groups or halogen atoms.

Preferably Y¹ is H₂C=CR¹⁴-CO-A⁸- where R¹⁴ is H or methyl and A⁸ is O or NH.

Preferred groups B¹ are alkanediyl, usually with linear alkyl chains and preferably having 2 to 12 carbon atoms, such as 2 or 3 carbon atoms.

Preferably Q is NR¹⁷₂ where R¹⁷ is C₁₋₁₂-alkyl. Preferably both R¹⁷'s are the same. Particularly useful results have been achieved where the groups R¹⁷ are C₁₋₄ alkyl, especially ethyl, methyl or isopropyl.

Either or both the hydrophobic and hydrophilic blocks may include comonomers, for instance to provide functionality, control over hydrophobicity, control over pH sensitivity, pKₐ or pK_{b} as the case may be, control over temperature sensitivity or as general diluents. For instance comonomers providing functionality may be useful to provide conjugation of pendant groups following polymerisation and/or self-assembled structuer formation, to targeting moieties, or to provide for conjugation between the biologically active molecule and the polymer. Alternatively, functional groups may allow for crosslinking of the polymer following construct formation, to confer increased stability on the construct structure.

Examples of suitable comonomers are compounds of the general formula VIII 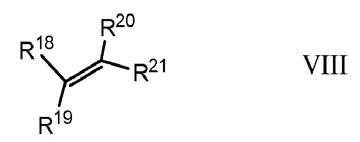 in which R¹⁸ is selected from hydrogen, halogen, C₁₋₄ alkyl and groups COOR²² in which R²² is hydrogen and C₁₋₄ alkyl;
R¹⁹ is selected from hydrogen, halogen and C₁₋₄ alkyl;
R²⁰ is selected from hydrogen, halogen, C₁₋₄ alkyl and groups COOR²² provided that R¹⁸ and R²⁰ are not both COOR²²; and
R²¹ is a C₁₋₁₀ alkyl, a C₁₋₂₀ alkoxycarbonyl, a mono-or di-(C₁₋₂₀ alkyl) amino carbonyl, a C₆₋₂₀ aryl (including alkaryl) a C₇₋₂₀ aralkyl, a C₆₋₂₀ aryloxycarbonyl, a C₁₋₂₀ -aralkyloxycarbonyl, a C₆₋₂₀ arylamino carbonyl, a C₇₋₂₀ aralkyl-amino, a hydroxyl or a C₂₋₁₀ acyloxy group, any of which may have one or more substituents selected from halogen atoms, alkoxy, oligo-alkoxy, aryloxy, acyloxy, acylamino, amine (including mono and di-alkyl amino and trialkylammonium in which the alkyl groups may be substituted), carboxyl, sulphonyl, phosphoryl, phosphino, (including mono- and di- alkyl phosphine and tri-alkylphosphonium), zwitterionic, hydroxyl groups, vinyloxycarbonyl and other vinylic or allylic substituents, and reactive silyl or silyloxy groups, such as trialkoxysilyl groups;
   or R²¹ and R²⁰ or R²¹ and R¹⁹ may together form -CONR²³CO in which R²³ is a C₁₋₂₀ alkyl group.

It is preferred for at least two of the groups R¹⁸, R¹⁹, R²⁰ and R²¹ to be halogen or, more preferably, hydrogen atoms. Preferably R¹⁸ and R¹⁹ are both hydrogen atoms. It is particularly preferred that compound of general formula X be a styrene-based or acrylic based compound. In styrene based compounds R²¹ represents an aryl group, especially a substituted aryl group in which the substituent is an amino alkyl group, a carboxylate or a sulphonate group. Where the comonomer is an acrylic type compound, R²¹ is an alkoxycarbonyl, an alkyl amino carbonyl, or an aryloxy carbonyl group. Most preferably in such compounds R²¹ is a C₁₋₂₀ -alkoxy carbonyl group, optionally having a hydroxy substituent. Acrylic compounds are generally methacrylic in which case R²⁰ is methyl.

Preferably the comonomer is a non-ionic comonomer, such as a C₁₋₂₄ alkyl(alk)-acrylate or -acrylamide, mono- or di- hydroxy-C₁₋₆-alkyl(alk)-acrylate, or acrylamide, oligo(C₂₋₃ alkoxy) C₂₋₁₈-alkyl (alk)-acrylate, or -acrylamide, styrene, vinylacetate or N-vinyllactam.

For optimum nanovesicle formation, the block copolymers should have controlled molecular weights. It is preferable for each of the blocks to have molecular weight controlled within a narrow band, that is, to have a narrow polydispersity. The polydispersity of molecular weight should, for instance, be less than 2.0, more preferably less than 1.5, for instance in the range 1.1 to 1.4.

Of course, in the preferred embodiment of this invention wherein one of the blocks has a pKₐ in the range 3.0 to 6.9, the blocks should be selected so that they have the requisite pKₐ value.

In one embodiment of this invention, the monomer from which the hydrophobic block is formed is 2-(diisopropylamino)ethyl methacrylate (DPA) or 2-(diethylamino)ethyl methacrylate (DEA). In another embodiment, the hydrophilic block is PMPC. Preferably, the copolymer is a PMPC-b-PDPA block copolymer.

Preferably, the block copolymer has general formula PMPCₘ-*b*-PDPAₙ,
wherein m is in the range 15-30 (for instance, 25) and n is 50 to 180 or 70 to 180, preferably 100 to 160, more preferably 120 to 160.

Typically, the hydrophobic block is not formed from 2-(dimethyl)ethyl methacrylate (DMA) monomers.

The block copolymer may be a simple A-B block copolymer, or may be an A-B-A or B-A-B block copolymer (where A is the hydrophilic block and B is the hydrophobic block). It may also be an A-B-C, A-C-B or B-A-C block copolymer, where C is a different type of block. C blocks may, for instance, comprise functional, e.g. cross-linking or ionic groups, to allow for reactions of the copolymer, for instance in the novel compositions. Crosslinking reactions especially of A-C-B type copolymers, may confer useful stability on nanovesicles. Cross-linking may be covalent, or sometimes, electrostatic in nature. Cross-linking may involve addition of a separate reagent to link functional groups, such as using a difunctional alkylating agent to link two amino groups. The block copolymer may alternatively be a star type molecule with hydrophilic or hydrophobic core, or may be a comb polymer having a hydrophilic backbone (block) and hydrophobic pendant blocks or *vice versa*. Such polymers may be formed by instance by the random copolymerisation of monounsaturated macromers and monomers.

It may be possible to synthesise the block copolymer by initial formation of a low polydispersity, low molecular weight initial block using control of initiator and chain transfer agent (which permanently terminates chain formation), with the initial block then being derivatised to act as a suitable radical initiator in a subsequent block forming step, by the technique described by Inoue *et al op*. *cit*.. Low polydispersity low molecular weight polymers which may be derivatised to form a substrate for the block polymerisation of second block are commercially available. Such polymers are, for instance, poly(alkylene oxides), poly(dimethyl siloxanes), polyimides, acrylic copolymers, etc. Some examples in which oligomers are derivatised to form initiator compounds for controlled radical polymerisation are described below. Preferably, the polymerisation of at least one of the blocks and preferably both the blocks is by controlled radical polymerisation for instance a living radical polymerisation process.

A living radical polymerisation process may be a group transfer radical polymerisation, for instance in which an N→O, or other carbon-, sulphur-, and oxygen-centered radical group is transferred from an initiator compound to a monomer. Preferably, however, the process is an atom transfer radical polymerisation process. Preferably such a process is used to form each block of the block copolymer.

In the atom or group transfer radical polymerisation process, the initiator has a radically transferable atom or group, and the catalyst comprises a transition metal compound and a ligand, in which the transition metal compound is capable of participating in a redox cycle with the initiator and dormant polymer chain, and the ligand is either any N-, O-, P- or S- containing compound which can coordinate with the transition metal atom in a σ-bond, or any carbon-containing compound which can coordinate with the transition metal in a π-bond, such that direct bonds between the transition metal and growing polymer radicals and not formed.

Preferably the radical initiator is of the general formula IX

R²⁴R²⁵R²⁶C-X² IX

where X² is selected from the group consisting of Cl, Br, I, OR²⁷, SR²⁸, SeR²⁸, OP(=O)R²⁸, OP(=O)(OR²⁸)₂, O-N(R²⁸)₂ and S-C(=S)N(R²⁸)₂, where R⁷ is alkyl of from 1 to 20 carbon atoms in which each of the hydrogen atoms may be independently replaced by halide, R²⁸ is aryl or a straight or branched C₁-C₂₀ alkyl group, and where an N(R²⁸)₂ group is present, the two R²⁸ groups may be joined to form a 5- or 6-membered heterocyclic ring; and

R²⁴, R²⁵ and R²⁶ are each independently selected from the group consisting of H, halogen, C₁-C₂₀ alkyl, C₃-C₈ cycloalkyl, C(=O)R²⁹, C(=O)NR³⁰R³¹, COCI, OH, CN, C₂-C₂₀ alkenyl, C₂-C₂₀ alkenyl oxiranyl, glycidyl, aryl, heterocyclyl, aralkyl, aralkenyl, C₁₋-C₆ alkyl in which from 1 to all of the hydrogen atoms are replaced with halogen, C₁-C₆ alkyl substituted with from 1 to 3 substituents selected from the group consisting of C₁-C₄ alkoxy, aryl, heterocyclyl, C(=O)R²⁹, C(=O)NR³⁰R³¹, -CR²⁵R²⁶X², oxiranyl and glycidyl;
where R²⁹ is alkyl of from 1 to 20 carbon atoms, alkoxy of from 1 to 20 carbon atoms, oligo(alkoxy) in which each alkoxy group has 1 to 3 carbon atoms, aryloxy or heterocyclyloxy any of which groups may have substituents selected from optionally substituted alkoxy, oligoalkoxy, amino (including mono- and di-alkyl amino and trialkyl ammonium, which alkyl groups, in turn may have substituents selected from acyl, alkoxycarbonyl, alkenoxycarbonyl, aryl and hydroxy) and hydroxyl groups; and

R³⁰ and R³¹ are independently H or alkyl of from 1 to 20 carbon atoms which alkyl groups, in turn may have substituents selected from acyl, alkoxycarbonyl, alkenoxycarbonyl, aryl and hydroxy, or R³⁰ and R³¹ may be joined together to form an alkanediyl group of from 2 to 5 carbon atoms, thus forming a 3- to 6-membered ring;
such that not more than two of R²⁴, R²⁵ and R²⁶ are H.

In the initiator of the general formula IX it is preferred that no more than one of R²⁴, R²⁵ and R²⁶, and preferably none, is hydrogen. Suitably at least one, and preferably both of R²⁴ and R²⁵ is methyl. R²⁶ is suitably a group CO-R²⁹ in which R²⁹ is preferably alkoxy of from 1 to 20 carbon atoms, oligo(alkoxy) in which each alkoxy group has 1 to 3 carbon atoms, aryloxy or heterocyclyloxy any of which groups may have substituents selected from optionally substituted alkoxy, oligoalkoxy, amino (including mono-- and di-alkyl amino and trialkyl ammonium, which alkyl groups, in turn may have substituents selected from acyl, alkoxycarbonyl, alkenoxycarbonyl, aryl and hydroxy) and hydroxyl groups.

Since any of R²⁴, R²⁵ and R²⁶ may comprise a substituent C²⁵R²⁶X², the initiator may be di-, oligo- or poly- functional, which may be of use to form A-B-A type copolymers or star polymers.

Selection of a suitable initiator is based on various considerations. Where the polymerisation is carried out in the liquid phase, in which the monomers are dissolved, it is preferable for the initiator to be soluble in that liquid phase. The initiator is thus selected for its solubility characteristics according to the solvent system which in turn is selected according to the monomers being polymerised.

Water-soluble initiators include, for instance the reaction product of monomethoxy-capped oligo(ethylene oxide) with 2-bromoisobutyryl bromide (OEGBr), 4-bromo-α-toluic acid or ethyl 2-bromopropanoic acid or 2-(N,N-dimethylamino) ethyl-2'-bromoisobutyrate.

The portion of the initiator -C-R²⁴R²⁵R²⁶ becomes joined to the first monomer of the growing polymer chain. The group X² becomes joined to the terminal unit of the polymer chain. Selection of a suitable initiator is determined in part by whether a terminal group having particular characteristics is required for subsequent functionality. Subsequent reactions of the product polymer are described below. The residue of the initiator at one or other end of the polymer may be reacted with biologically active moieties, such as targetting groups. Alternatively the initiator itself may comprise a group conferring useful targetting or other biological activity in the product polymer. Alternatively the initiator may comprise a highly hydrophobic group, which is polymeric and which may consequently form the part of the hydrophobic block. Suitable hydrophobic polymers which may be converted into initiators are, for instance, poly(propylene oxide) and poly(dimethyl siloxane). In an atom or group radical transfer polymerisation process the transition metal compound which comprises a component of the catalyst is Mₜ⁺X³ₙ, where: Mₜⁿ⁺ may be selected from the group consisting of Cu¹⁺, Cu²⁺, Fe²⁺, Fe³⁺, Ru²⁺, Ru³⁺, Cr²⁺, Cr³⁺, Mo²⁺, Mo³⁺, W²⁺, W³⁺, Mn²⁺, Mn³⁺, Mn⁴⁺, Rh³⁺, Rh⁴⁺, Re²⁺, Re³⁺, Co⁺, Co²⁺, Co³⁺, V²⁺, V³⁺, Zn⁺, Zn²⁺, Ni²⁺, Ni³⁺, Au⁺, Au²⁺, Ag⁺ and Ag²⁺;

X³ is selected from the group consisting of halogen, C₁-C₆-alkoxy, (SO₄)_{½}, (PO₄)_{1/3}, (R³²PO₄)½, (R³²₂PO₄), triflate, hexafluorophosphate, methanesulphonate, arylsulphonate, CN and R³³CO₂, where R³² is aryl or a straight or branched C₁₋₂₀ alkyl and R³³ is H or a straight or branched C₁-C₆ alkyl group which may be substituted from 1 to 5 times with a halogen; and
n is the formal charge on the metal (0 ≤ n ≤7).

Preferably X³ is halide, most preferably chloride or bromide. Particularly suitable transition metal compounds are based on copper or ruthenium, for instance CuCl, CuBr or RuCl₂.

When the hydrophilic block is a polyalkylene glycol, for instance PEG, the hydrophobic block is typically polymerised using a PEG-Br initiator. PEG-PDPA copolymer can be synthesised from PDPA and PEG-Br in this manner.

In the catalyst, the ligand is preferably selected from the group consisting of:
a) compounds of the formulas:

   R³⁴-Z-R³⁵

   and

   R³⁴-Z-(R³⁶-Z)ₘ-R³⁵

   where:
   R³⁴ and R³⁵ are independently selected from the group consisting of H, C₁-C₂₀ alkyl, aryl, heterocyclyl and C₁-C₆ alkoxy, C₁-C₄ dialkylamino, C(=O)R³⁷ and A⁷C(=O)R⁴⁰, where A⁷ may be NR⁴¹ or O; R³⁷ is alkyl of from 1 to 20 carbon atoms, aryloxy or heterocyclyloxy; R⁴⁰ is H, straight or branched C₁-C₂₀ alkyl or aryl and R⁴¹ is hydrogen, straight or branched; C₁₋₂₀-alkyl or aryl; or R³⁴ and R³⁵ may be joined to form, together with Z, a saturated or unsaturated ring;
   Z is O, S, NR⁴² or PR⁴², where R⁴² is selected from the same group as R³⁴ and R³⁵, and where Z is PR⁴², R⁴² can also C₁-C₂₀ alkoxy or Z may be a bond, CH₂ or a fused ring, where one or both of R³⁴ and R³⁵ is heterocyclyl,
   each R³⁶ is independently a divalent group selected from the group consisting of C₁-C₈ cycloalkanediyl, C₁-C₈ cycloalkenediyl, arenediyl and heterocyclylene where the covalent bonds to each Z are at vicinal positions or R³⁶ may be joined to one or both of R³⁴ and R³⁵ to formulate a heterocyclic ring system; and;
   m is from 1 to 6;
b) CO;
c) porphyrins and porphycenes, which may be substituted with from 1 to 6 halogen atoms, C₁₋₆ alkyl groups, C₁₋₆-alkoxy groups, C₁₋₆ alkoxycarbonyl, aryl groups, heterocyclyl groups, and C₁₋₆ alkyl groups further substituted with from 1 to 3 halogens;
d) compounds of the formula R⁴³R⁴⁴C(C(=O)R⁴⁵)₂, where R^{45 is} C₁₋₂₀ alkyl, C₁₋₂₀ alkoxy, aryloxy or heterocyclyloxy; and each of R⁴³ and R⁴⁴ is independently selected from the group consisting of H, halogen, C₁₋₂₀ alkyl, aryl and heterocyclyl, and R⁴³ and R⁴⁴ may be joined to form a C₁₋₈ cycloalkyl ring or a hydrogenated aromatic or heterocyclic ring, of which the ring atoms may be further substituted with 1 to 5 C₁₋₆ alkyl groups, C₁₋₆ alkoxy groups, halogen atoms, aryl groups, or combinations thereof; and
e) arenes and cyclopentadienyl ligands, where said cyclopentadienyl ligand may be substituted with from one to five methyl groups, or may be linked through and ethylene or propylene chain to a second cyclopentadienyl ligand.

Selection of a suitable ligand is, for instance, based upon the solubility characteristics and/or the separability of the catalyst from the product polymer mixture. Generally it is desired for the catalyst to be soluble in a liquid reaction mixture, although under some circumstances it may be possible to immobilise the catalyst, for instance on a porous substrate. For the preferred process, which is carried out in the liquid phase, the ligand is soluble in a liquid phase. The ligand is generally a nitrogen containing ligand. The preferred ligand may be a compound including a pyridyl group, such as bipyridine, or a compound including a pyridyl group and an imino moiety, such as 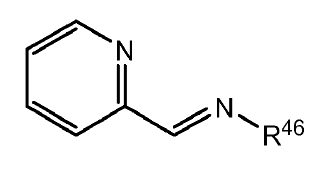 where R⁴⁶ is a suitable alkyl group, the substituent being variable and adaptable to confer desired solubility characteristics, triphenylphosphine or 1,1,4,7,10,10-hexamethyl-triethylene tetramine.

Such ligands are usefully used in combination with copper chloride, copper bromide and ruthenium chloride transition metal compounds as part of the catalyst. The ratio of metal compound and ligand in the catalyst should be approximately stoichiometric, based on the ratios of the components when the metal ion is fully complexed. The ratio should preferably be in the range 1:(0.5 to 2) more preferably in the range 1:(0.8:1.25). Preferably the range is about 1:1.

In the living radical polymerisation process, the catalyst may be used in amounts such that a molar equivalent quantity as compared to the level of initiator is present. However, since catalyst is not consumed in the reaction, it is generally not essential to include levels of catalyst as high as of initiator. The ratio of catalyst (based on transition metal compound) to initiator is preferably in the range 1:(1 to 50), more preferably in the range 1:(1 to 10).

Whilst the polymerisation reaction may be carried out in the gaseous phase, it is more preferably carried out in the liquid phase. The reaction may be heterogeneous, that is comprising a solid and a liquid phase, but is more preferably homogeneous. Preferably the polymerisation is carried out in a single liquid phase. Where the monomer is liquid, it is sometimes unnecessary to include a non-polymerisable solvent. More often, however, the polymerisation takes place in the presence of a non-polymerisable solvent. The solvent should be selected having regard to the nature of the zwitterionic monomer and any comonomer, for instance for its suitability for providing a common solution containing both monomers. The solvent may comprise a single compound or a mixture of compounds.

It has been found that, especially where the zwitterionic monomer is MPC, that it is may be desirable to include water in the polymerisation mixture. In such processes water should be present in an amount in the range 10 to 100% by weight based on the weight of ethylenically unsaturated monomer. Preferably the total non-polymerisable solvent comprised 1 to 500% by weight based on the weight of ethylenically unsaturated monomer. It has been found that the zwitterionic monomer and water should be in contact with each other for as short a period as possible prior to contact with the initiator and catalyst. It may be desirable therefore for all the components of the polymerisation other than the zwitterionic monomer to be premixed and for the zwitterionic monomer to be added to the premix as the last additive.

It is often desired to copolymerise MPC or other zwitterionic monomer with a comonomer which is insoluble in water. In such circumstances, a solvent or co-solvent (in conjunction with water) is included to confer solubility on both MPC and the more hydrophobic monomer. Suitable organic solvents are ethers, esters and, most preferably, alcohols. Especially where a mixture of organic solvent and water is to used, suitable alcohols are C₁₋₄ -alkanols. Methanol is found to be particularly suitable in the polymerisation process of the invention.

The process may be carried out at raised temperature, for instance up to 60 to 80 °C. However it has been found that the process proceeds sufficiently fast at ambient temperature.

The living radical polymerisation process has been found to provide polymers of zwitterionic monomers having a polydispersity (of molecular weight) of less than 1.5, as judged by gel permeation chromatography. Polydispersities in the range 1.2 to 1.4 for the or each block are preferred.

### Self-Assembled Construct Preparation

An advantage of the present invention where one of the blocks is pH sensitive, is that the self-assembled constructs may be loaded using a pH change system. In such a process, copolymer is dispersed in aqueous liquid in ionised form, in which it solubilises at relatively high concentrations without forming constructs. Subsequently the pH is changed such that some or all of the ionised groups become deprotonated so that they are in non-ionic form. At the second pH, the hydrophobicity of the block increases and constructs are formed spontaneously.

The method of forming self-assembled constructs associated with imaging agent (generally encapsulated in their core) may involve the following steps:
(i) dissolving the amphiphilic copolymer in an aqueous medium;
(ii) acidifying the composition formed in step (i);
(iii) adding the imaging agent to the composition; and
(iv) raising the pH to around neutral.

This method preferably comprises a further, preliminary step wherein the amphiphilic copolymer is dissolved in an organic solvent in a reaction vessel and the solvent is then evaporated to form a film on the inside of the reaction vessel.

In more detail, constructs are typically prepared by dissolving copolymer in an organic solvent, such as a 2:1 chloroform:methanol mix in a glass container. If the imaging agent is water-insoluble, it is typically added at this stage. Solvent can be evaporated under vacuum leaving a copolymeric film deposited on the walls of the container. The film is then re-hydrated with an aqueous solution, for instance using phosphate buffer saline. The pH of the resultant suspension is decreased to a pH of around 2, to solubilise the film, and then increased, slowly to around a pH of 6. Once the pH has reached this value, a water soluble imaging agent may be added. The pH is then increased to around neutral, to encapsulate the imaging agent. The dispersion may then be sonicated and extruded, for instance using a bench top extruder. UV spectroscopy may be used to calculate encapsulation efficiency, using techniques well known in the art.

An alternative method for forming self-assembled constructs with encapsulated imaging agent may involve simple equilibration of the agent and empty polymer constructs in water. The empty constructs may be pre-formed by the method detailed above, or other methods known in the art. For instance agent may be contacted in solid form with an aqueous dispersion of polymer constructs and incubated, optionally with shaking, to solubilise the imaging agent in the dispersed constructs. Alternatively, agent dissolved in organic solvent may be emulsified into an aqueous dispersion of polymer constructs, whereby solvent and agent become incorporated into the core of the constructs, followed by evaporation of solvent from the system.

Generally, 0.01% to 10% (w/w) of imaging agent is mixed with copolymer in the methods described above.

### Imaging Agent

In the method according to the first aspect of this invention, the imaging agent is any compound which may be used to label the cells and allow them to be monitored. Preferably, the imaging agent is a fluorescent label, and the cells are imaged by monitoring the fluorescence emitted from the label.

The imaging agent is typically associated with the self-assembled constructs *via* physical or chemical interaction, such as electrostatic or hydrophobic attraction. Usually the imaging agent is not covalently bound to the self-assembled constructs. The location of the imaging agent is generally determined by its hydrophobic or hydrophilic nature. The agent may be associated with the interior of the membrane, or with the external surface of membrane-enclosed self-assembled constructs. However, preferably the imaging agent is encapsulated within the aqueous core of the construct, which is preferably a nanovesicle.

Several methods known in the art can be used to determine that the imaging agent is encapsulated within the nanovesicles. Transition Electron Microscopy for instance provides images from which the position of the imaging agent, such as a fluorescent dye, can be located.

Generally, after the constructs are taken up by the live cells in the method according to the first aspect of this invention, they dissociate and release imaging agent into the cell cytosol. This then allows the imaging agent to target a specific cellular structure, as further detailed below.

A suitable label for use in the present invention is any label which fluoresces when excited with electromagnetic radiation, and can be associated with the self-assembled structures. Typically, the fluorescent label is encapsulated within the aqueous core of vesicles. However, when the fluroescent label is hydrophobic, more typically it is associated with the hydrophobic membrane. Fluorescent dyes, such as rhodamine fluorescein, BODIPY® and NBD are particularly suitable.

Preferably, the imaging agent is a biomolecule, or is derived from a biomolecule. By "biomolecule", is meant a chemical compound that naturally occurs in living organisms. A biomolecule may be, for instance, a nucleic acid, protein, carbohydrate or lipid. Biomolecules can be modified to enable them to act as imaging agents for instance, by covalent or other attachment of a fluorescent dye. The imaging agent can be selected to target a particular cellular structure. Indeed, in the composition according to the second aspect of this invention, the imaging agent is a biomolecule capable of targeting a specific cellular structure. Imaging agents based on phospholipids, for instance, are known to target cellular membranes. A suitable example is fluorescein DHPE (N-(fluorescein-5-thiocarbamoyl)-1,2-dihexadecanoyl-sn-glycero-3-phosphoethanolamine triethylammonium salt), which is known to target bilayer membranes, such as the cell membrane. BODIPY® FL C5-ceramide, may similarly be used to target lipid membranes. In the method of the invention, nanovesicles associated with these molecules are taken up by cells, and the delivery of these molecules to either the cell membrane, or internal membranes can be monitored. Other examples of suitable biomolecules are sphyngolipids and cholesterol. Phospholipids, sphyngolipids and cholesterol molecules target internal and external components of the plasma membrane and can be used to study the formation and dynamics of lipid rafts, as well as the morphology of the cell and its organelles.

NBD cholesterol can be used to monitor the movement of cholesterol in a cell, since NBD cholesterol mimics cholesterol, and the cell treats it as such. Other dyes may be selected by the skilled person to target particular organelles in the cell, such as the Golgi or the nucleus. Mitosis, for instance, can be monitored using dyes which stain the nucleus.

Other imaging agents, which may have utility in the present invention, include fluorescent proteins (for instance, green fluorescent protein, GFP), labelled nucleic acids, labelled toxins (for instance, phalloidin for actin staining) and labelled ion indicators.

Other compounds, in addition to a fluorescent label, may be associated with the self-assembled constructs. DNA, for instance, may be delivered to cells along with a imaging agent in the method of the present invention. The imaging agent is preferably attached to the other compound, but this is not essential.

### Cell Monitoring

The method of monitoring cells may comprise monitoring dynamic processes such as cell signalling, excitability, growth and metabolic transport. The method may be used in cell viability assays, cell adhesion, cell migration and cell-cell fusion studies. The method is typically performed *in vitro*.

When the imaging agent is a fluorescent label, the fluorescence emission from cells can be measured using confocal laser scanning microscopy, inverted phase contrast, epifluorescent microscopy, flow cytometry and/or spectroscopic techniques. The cells may be observed in 2 or 3 dimensions, for instance using immersion objective lenses in confocal laser scanning microscopy.

Typically, in the method of monitoring, cells are monitored for at least one day, sometimes at least one week. Cells may be monitored, in some embodiments, for at least 2 weeks, for instance 3 weeks. During this time, the cells may be observed and/or fluorescence intensity measurements taken at least once a day, more typically at least every 6 hours.

Generally the cells are imaged on two or more occasions. However, in some embodiments, just one image may be taken and a static picture of the cells obtained.

In different embodiments, monitoring is performed over a shorter period of time, for instance, 2 to 3 days, and observations/measurements are taken more frequently, for instance, every 3 hours. Alternatively, the period of monitoring may be much shorter, e.g. up to one day.

### Cells

In step (i) of the method according to the invention, the cells are contacted with the self-assembled constructs associated with imaging agent. Typically, a known volume of aqueous dispersion of constructs (for instance, 5-20mg/ml in PBS) is added to the cells in their culture media.

In the method of the present invention, after step (i), the cells are typically incubated for a period of time of at least 10 hours, preferably at least 24 hours. Step (i) allows the cells to take up the self-assembled constructs and optionally, release imaging agent into the cell. However, in different embodiments of the invention, this incubation step can be much shorter, for instance around 5 minutes to 2 hours. The incubation step should be at least 5 minutes, preferably at least 10 minutes, most preferably at least 30 minutes long. The live cells are generally cultured in a nutrient medium both before and during the method of monitoring. The nutrient medium is preferably aqueous and comprises the nutrients essential for the viability of the cells.

The cells may be human or animal cells, including primary cells, cancer cells and stem cells.

The method of monitoring may be the monitoring of cell division. During cell division, the fluorescent label will become distributed between parent and daughter cells, thereby reducing the fluorescence intensity observed from each individual cell monitored.

The migration of cells on a 3D-scaffold may be monitored.

The invention will now be illustrated by some Examples, which refer to the following figures:
Figure 1 shows the fluorescein DHPE phospholipid chemical formula and confocal laser scanning micrographs showing live Human Dermal Fibroblast (HDF) cells after phospholipid vesicle-mediated delivery;
Figure 2 shows the BODIPY® FL C5-ceramide formula and confocal laser scanning micrographs showing live HDF cells after phospholipid vesicle-mediated delivery;
Figure 3 shows the NBD-cholesterol chemical formula and confocal laser scanning micrographs showing live HDF cells after cholesterol vesicle-mediated delivery;
Figure 4 shows HDFs treated with both rhodamine octadecyl ester loaded vesicles and CellTracker^{™};
Figure 5 shows the data in Figure 4 normalized to the initial fluorescence intensity;
Figure 6 shows MTT assay data, indicating the cell viability of HDF after exposure to Rhodamine loaded vesicles and CellTracker^{™}.
Figure 7 shows human dermal fibroblasts (HDFs) stained with rhodamine encapsulated vesicles;
Figure 8 shows human dermal fibroblasts (HDFs) stained with vesicles and monitored using fluorescence microscopy; and
Figure 9 shows HDFs stained with vesicles after being cultured statically in DMEM for 10 days.

### EXAMPLES

### Example A: ATRP Synthesis of the PMPC₂₅-PDPA₇₀ Diblock Copolymer

In a typical ATRP procedure, a Schlenk flask with a magnetic stir bar and a rubber septum was charged with Cu(I)Br (25.6 mg, 0.178 mmol) and MPC (1.32 g, 4.46 mmol). ME-Br initiator (50.0 mg, 0.178 mmol) and bpy ligand (55.8 mg, 0.358 mmol) were dissolved in methanol (2 mL), and this solution was deoxygenated by bubbling N₂ for 30 minutes before being injected into the flask using a syringe. The [MPC]: [ME-Br]: [CuBr]: [bpy] relative molar ratios were 25: 1: 1: 2 and the reaction was carried out under a nitrogen atmosphere at 20°C. After 65 minutes, a deoxygenated mixture of DPA (2.67 g, 12.5 mmol) and methanol (3 mL) were injected into the flask. After 48 h, the reaction solution was diluted by addition of isopropanol (about 200 mL) and then passed through a silica column to remove the catalyst.

**Table 1: Properties of the PMPCPDPA diblock copolymer**

| **Copolymer composition by ¹H NMR in D₂O/DC1 at pH 2** | ***M*_{n,GPC}** | ***M*_{w}/*M*ₙ** | **TEM Morphologies** |
|---|---|---|---|
| PMPC₂₅₋PDPA₇₀ | 32900 | 1.16 | vesicles + micelles |

### Example B: PEO-PDPA Diblock Copolymer

The procedure is based on that followed Vamvakaki et al in Macromolecules; 1999; 32(6) pp 2088-2090.

The monohydroxy-capped poly(ethylene oxide) (PEO) was donated by Inspec U.K. GPC analyses gave Mw/Mn's of 1.10 for PEO; degrees of polymerization were either 22 or 45 for PEO. In a typical synthesis, PEO (5.0 g) dissolved in 100 mL of dry THF was added to a round-bottomed flask under dry nitrogen. Potassium naphthalene (2.50 mmol) in THF was added via a double-tipped needle, and the reaction solution was stirred at 30°C for 1-2 h to form the alcoholate macro-initiator. Freshly distilled tertiary amine methacrylate (5-15 mL) was added, and the polymerization was allowed to proceed for 4 h prior to quenching with methanol. In some cases the polymerizations were conducted at 35 or 50°C. Solvent was removed under vacuum, the copolymer was redissolved in dilute HCl, and the water-insoluble naphthalene was removed by filtration. PEG₁₁₃-PDPA₇₁ and PEG₁₀-PDPA₃₀ were described in high yields (95-100%) with good control over copolymer molecular weight.

### General Methods

### Vesicles Preparation

PMPC-PDPA copolymer was added to a glass vial and dissolved in a solution of 2:1 chloroform: methanol, at a concentration of 4 mg/ml. The solvent was evaporated under vacuum, resulting in a copolymeric film deposited on the walls of the vial. The copolymer film was sterilized in an autoclave and then rehydrated under sterile conditions using phosphate buffer saline (100 mM PBS) to form a 5mg/ml copolymer suspension. The pH of this suspension was dropped to pH 2 to solubilise the film again and the pH was increased to neutral pH. The dispersion was sonicated and extruded using a LiposoFast with a 200nm filter membrane.

### Amphiphilic and Hydrophobic Fluorescent Marker Encapsulation

The following dyes were used: octadecyl ether rhodamine B, (N-(fluorescein-5-thiocarbamoyl)-1,2-dihexadecanoyl-sn-glycero-3-phosphoethanolamine, triethylammonium salt (Fluorescein DHPE), N-(4,4-difluoro-5, 7-dimethyl-4-bora-3a, 4a-diaza-s-indacene-3-pentanoyl) sphingosine (BODIPY FL C5-ceramide), or 22-(N-(7-nitrobenz-2-oxa-1,3-diazol-4-yl)amino)-23,24-bisnor-5-cholen-3-ol (NBD cholesterol). These were dissolved in chloroform to form a 0.2 mg/ml solution. Aliquots of this solution were added to the sample of PMPC-PDPA copolymer dissolved in 2:1 chloroform/methanol. The vesicles were then prepared by film rehydration and sonication, as described above.

### Cell Culture

Primary human dermal fibroblasts (HDF) were isolated from skin obtained from abdominoplasty or breast reduction operations (according to local ethically approved guidelines, NHS Trust, Sheffield, UK). Primary cultures of fibroblasts were established as previously described (Ralston, D.R, et a/; Br. J. Dermatol. 1999, 140,605). Briefly, the epidermal layer of the skin was removed by trypsinisation and the remaining dermal layer was washed in PBS. The dermis was then minced using surgical blades and incubated in 0.5 % (w/v) collagenase A at 37°C overnight in a humidified CO₂ incubator. A cellular pellet was collected from the digest and cultured in DMEM (Sigma, UK) supplemented with 10% (v/v) foetal calf serum, 2 mM L-glutamine, 100 IU/ml penicillin, 100 mg/ml streptomycin and 0.625 mg/ml amphotericin B. Cells were sub-cultured routinely using 0.02 % (w/v) EDTA and used for experimentation between passages (replication cyles) 4 and 8.

### Flow Cytometry

The flow cytometry machine provides a cell counting and viability assay. During the data collection the machine draws samples from the sample loader through the flow cell, where a laser excites the sample. Light from the sample is scattered then collected by a photodiode and two photomultipliers (PM) where it is converted into data. The system uses the light collected by the photodiode, called forward scatter (FSC), as an indicator of particle size. It incorporates both the particles' cross-sectional diminution and refractive index. The first PM (PM1) identifies all events with fluorescence centered at 580 nm, the second one all the events with fluorescence centered at 675 nm. The data is then sent to the software for processing and analyzing.

### Example 1: Delivery of Fluorescent Molecules with PMPC₂₅-PDPA₇₀

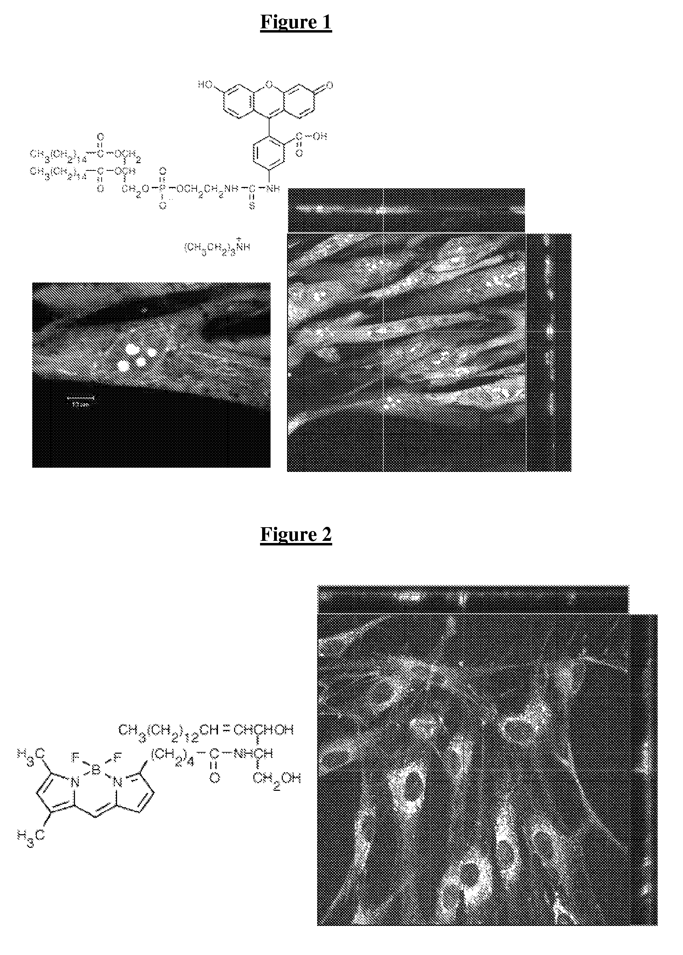

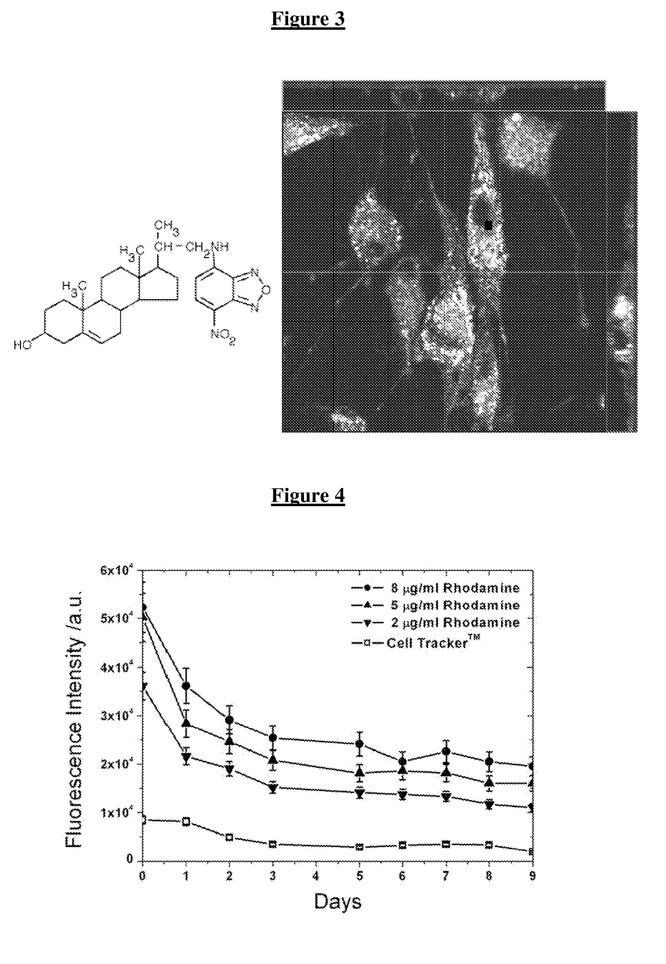

Figure 1 shows confocal laser scanning micrographs of live HDF cells after phospholipid vesicle-medicated delivery of the fluorescein DHPE. The bright spot within the cells are believed to be the cell nucleoli. These subcellular organelles contain chromatin and DNA and have a morphology that changes remarkably during the different cell phase. Each particular micrograph shows cells after the mitosis phase. Figures 2 and 3 show the results of using other fluorescent labels.

### Example 2: Cell labelling using Cell Tracker^{™} (Comparative)

After being cultured to 80-90% confluence in T25 tissue culture flasks and washed thoroughly with serum free DMEM medium, HDFs were incubated in prewarmed (37°C) fluorescent probe Cell Tracker^{™} Red CMTPX (C34552) (Invitrogen Ltd, UK) solution (0.5-25µM) prepared in serum free DMEM medium for 15-45 minutes at 37°C. This was followed by removing the Cell Tracker^{™} reagent, washing the cells thoroughly with prewarmed DMEM medium (with serum) and incubating the cells in DMEM medium for at least 30 minutes at 37°C. Then the cells were trypsinized, centrifuged to a pellet and used for either 2D or 3D cell culture. During or after cell culture, the fluorescently labelled cells were observed under epifluorescent microscopy or confocal laser scanning microscopy.

In figure 4, the fluorescence intensity was measured at different culture times for the rhodamine vesicles staining at different concentrations and the CellTracker^{™}. The data show that the vesicle mediated staining facilitates a large amount of fluorescent marker delivery and consequently higher fluorescence intensity than CellTracker^{™}.

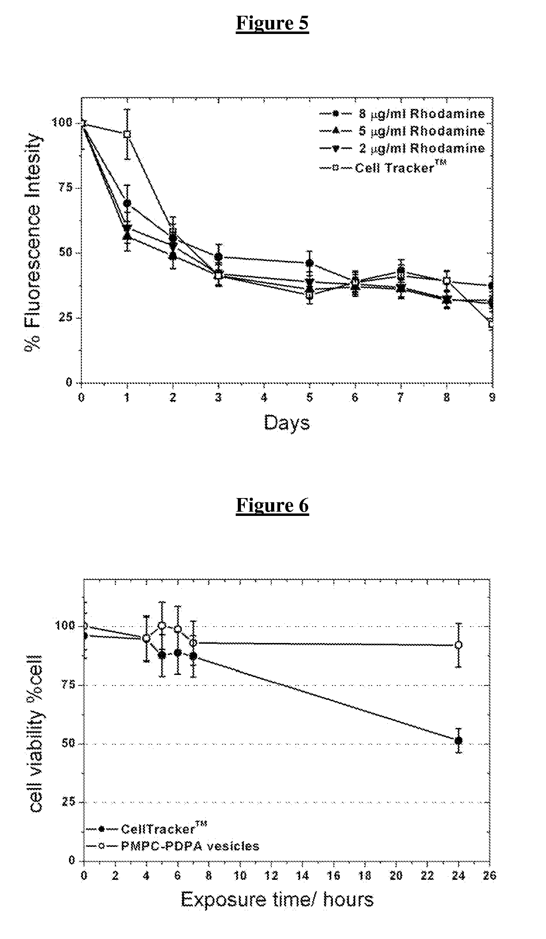

The data in Figure 5 show that rhodamine and CellTracker^{™} stay within the cell for the same period of time allowing cell tracking up to 9 days.

Figure 6 shows MTT assay data, indicating the cell viability of HDF after exposure to Rhodamine loaded vesicles and CellTracker^{™}. In the MTT assay, yellow MTT (3-(4,5-dimethylthiazol-2-yl)-2, 5-diphenyltetrazolium bromide, a tetrazole) is reduced to purple formazan in the mitochondria of living cells. A solubilisation solution (dimethyl sulfoxide, an acidified ethanol solution, or a solution of the detergent sodium dodecyl sulphate in dilute hydrochloric acid) is added to dissolve the insoluble purple formazan product into a coloured solution. The absorbance of this coloured solution can be quantified by measuring at a certain wavelength (usually between 500 and 600nm) by a spectrophotometer. The absorption maximum is dependent on the solvent employed.

This reduction takes place only when mitochondrial reductase enzymes are active, and therefore conversion can be directly related to the number of viable (living) cells. When the amount of purple formazan produced by cells treated with an agent is compared with the amount of formazan produced by untreated controlled cells, the effectiveness of the agent in causing death of cells can be deduced, through the production of a dose-response curve.

### Example 3: Cell seeding using fibrin clot

A fibrin clot was used to seed cells at specific areas for some of the 2D and all the 3D culture experiments. Briefly, a cell suspension was prepared by mixing fibrinogen (3.5mg/ml), thrombin (10 units/ml) and HDFs (10⁵ cells per ml of fibrin fibrinogen) together. Tiny drops (10-20µm) of the cell suspension were quickly transferred to specific areas of either 2D or 3D culture systems. After being incubated at 37°C for 20-40 minutes, the fibrinogen polymerised and the cells were embedded in the fibrin clot. Cell culture was subsequently carried out by submerging the cells and/or the 3D scaffolds in DMEM medium. In this research, whether the HDFs were stained (with either vesicles or Cell Tracker^{™}) or not, the cell densities in all the cell suspensions were constant (10⁵ cells per ml of fibrin fibrinogen).

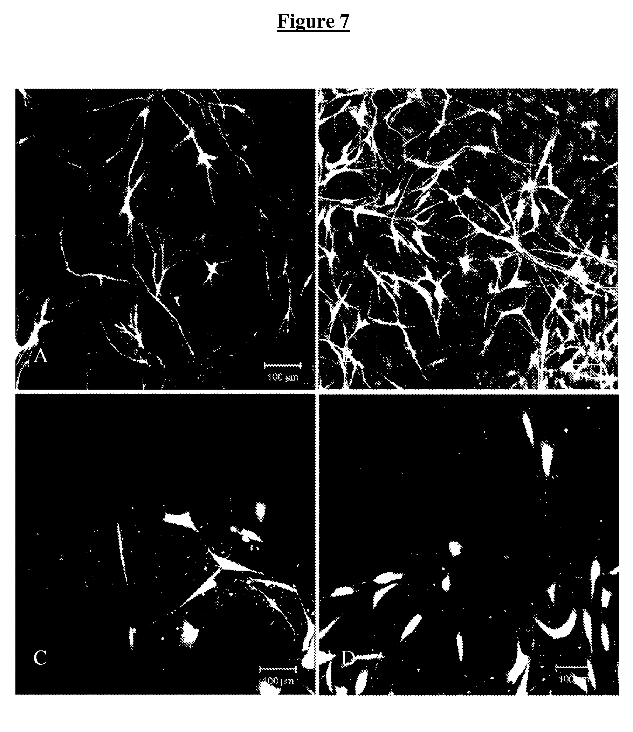

In Figure 7, the HDFs were stained with rhodamine encapsulated vesicles for epifluorescent microscopy and or confocal microscopy after being cultured for (A, C) 6 days and (B, D) 12 days in DMEM media with 10% FCS. The fluorescent (Red) micrographs show HDFs at (A, B) in the initial cell seeding fibrin clot and (C, D) at the migration front line outside the fibrin clot. Scale bars are 100µm.

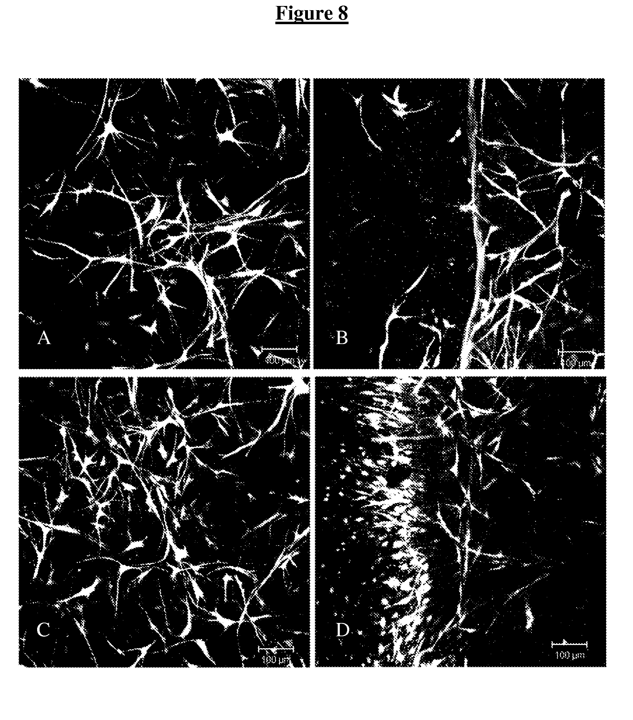

In Figure 8, the HDFs were seeded at one end of the pre-cast fibrin gel using fibrin clot cell seeding method in 6-well tissue culture plate. These were stained with vesicles and monitored using fluorescent microscopy after being cultured statically in DMEM media with 10% FCS for (A, B) 7 days and (C, D) 14 days respectively. Fluorescent micrographs show HDFs (A, C) in the initial cell seeding fibrin clot, (B, D) in border area between the initial cell seeding area and the pre-cast gel. Scale bars are 100µm.

### Example 4: 3D cell culture using fibrin clot

A slight modification was made to the previously developed 3D cell culture system using fibrin clot (Sun, T et al; Biomaterials, 27, 3459-3465, 2006). Briefly, plasminogen-free bovine fibrinogen (3.5mg/ml) dissolved in serum free DMEM medium was mixed thoroughly with bovine thrombin (10IU/ml), and then quickly transferred into a silicon mould attached to 6-well tissue culture plate to cast a thin fibrin gel with the thickness of 0.2-0.5mm. After the fibrin matrix gelled within 20-40 minutes, HDFs were seeded specifically on one end of the pre-cast gel using the cell seeding method described earlier. Cell culture was then carried out by submerging the gel and the cells in DMEM medium at 37°C, 95% air/5% CO₂. During cell culture in the 3D fibrin clot scaffold, the media were changed 2-3 times a week. To monitor the performance of the culture systems and/or investigate cell-cell cell-scaffold interactions at different culture time points, the 6 well plates were taken out of the incubator and image analysis of the cells was performed from the bottom of the plate using inverted phase-contrast or epifluorescent microscopy. For confocal laser scanning microscopy, immersion objective lenses were used to monitor the cell cultured in 2D or 3D environments directly.

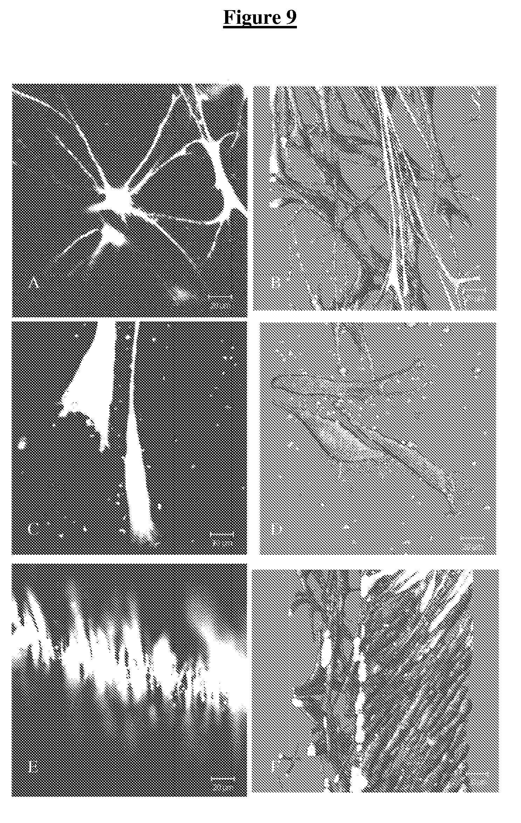

In Figure 9 human dermal fibroblasts (HDFs) were seeded at one end of the pre-cast fibrin gel using fibrin clot cell seeding method in 6-well tissue culture plate. These were stained with vesicles (Red), SYTO 9 dye (Green), and then analyzed using laser scanning confocal microscopy after being cultured statically in DMEM media with 10% FCS for 10 days. Fluorescent micrographs and 3D volume reconstructions show some of the HDFs in (A, B) the initial cell seeding fibrin clot, (B, C) migration front line with singular cells in the pre-cast gel and (E, F) some area with higher cell density in the pre-cast gel. Scale bars are 20µm.

## Claims

1. A method of monitoring live cells, comprising the steps of:
(i) contacting the cells with self-assembled constructs associated with an imaging agent;
(ii) incubating the cells;
(iii) imaging the live cells;
wherein in step (i), at least some of the constructs are taken up into the cells;
and wherein the constructs comprise an amphiphilic block copolymer having a hydrophilic and hydrophobic block.

2. A method according to claim 1 wherein the constructs are nanovesicles, micelles or a mixture of nanovesicles and micelles.

3. A method according to claim 1 or 2 wherein one of the blocks, preferably the hydrophobic block comprises pendant groups which have a pKₐ in the range 3.0 to 6.9.

4. A method according to claim 3, wherein the pendant groups have a pKₐ in the range 4.0 to 6.9.

5. A method according to any preceding claim wherein the pendant groups are tertiary amines.

6. A method according to any preceding claim wherein the hydrophilic block is a polyalkylene oxide, preferably polyethylene oxide.

7. A method according to any of claims 1 to 5 wherein the hydrophilic block comprises a zwitterionic monomer.

8. A method according to claim 7 in which the zwitterionic monomer has the general formula Y B X I
in which Y is an ethylenically unsaturated group selected from H₂C=CR-CO-A-, H₂C=CR-C₆H₄-A¹-, H₂C=CR-CH₂A², R²O-CO-CR=CR-CO-O, RCH=CH-CO-O-, RCH=C(COOR²)CH₂-CO-O, 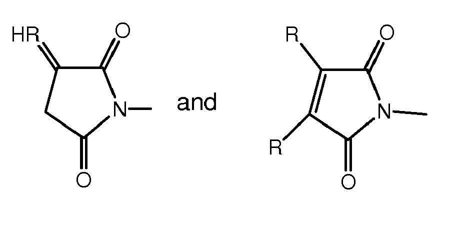
A is -O- or NR¹;
A¹ is selected from a bond, (CH₂)_{I}A² and (CH₂)_{I} SO₃⁻ in which I is 1 to 12;
A² is selected from a bond, -O-, O-CO-, CO-O, CO-NR¹-, -NR¹-CO, O-CO-NR¹-, NR¹-CO-O-;
R is hydrogen or C₁₋₄ alkyl;
R¹ is hydrogen, C₁₋₄₋ alkyl or BX;
R² is hydrogen or C₁₋₄ alkyl;
B is a bond, or a straight branched alkanediyl, alkylene oxaalkylene, or alkylene (oligooxalkylene) group, optionally containing one or more fluorine substituents;
X is a zwitterionic group.

9. A method according to claim 8 in which X is a group of the general formula II 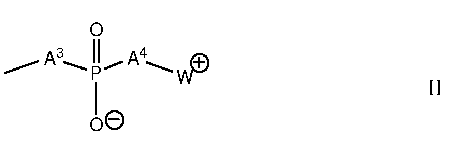 in which the moieties A³ and A⁴, which are the same or different, are -O-, -S-, -NH- or a valence bond, preferably -O-, and W⁺ is a group comprising an ammonium, phosphonium or sulphonium cationic group and a group linking the anionic and cationic moieties which is preferably a C₁₋₁₂-alkanediyl group,
preferably in which W⁺ is a group of formula
-W¹-N⁺R³₃, -W¹-P⁺R⁴₃, -W¹-S⁺R⁴₂ or -W¹-Het⁺ in which:
W¹ is alkanediyl of 1 or more, preferably 2-6 carbon atoms optionally containing one or more ethylenically unsaturated double or triple bonds, disubstituted-aryl (arylene), alkylene arylene, arylene alkylene, or alkylene aryl alkylene, cycloalkanediyl, alkylene cycloalkyl, cycloalkyl alkylene or alkylene cycloalkyl alkylene, which group W¹ optionally contains one or more fluorine substituents and/or one or more functional groups; and
either the groups R³ are the same or different and each is hydrogen or alkyl of 1 to 4 carbon atoms, preferably methyl, or aryl, such as phenyl, or two of the groups R³ together with the nitrogen atom to which they are attached form an aliphatic heterocyclic ring containing from 5 to 7 atoms, or the three groups R³ together with the nitrogen atom to which they are attached as heteroaromatic ring having 5 to 7 atoms, either of which rings may be fused with another saturated or unsaturated ring to form a fused ring structure containing from 5 to 7 atoms in each ring, and optionally one or more of the groups R³ is substituted by a hydrophilic functional group, and
the groups R⁴ are the same or different and each is R³ or a group OR³, where R³ is as defined above; or
Het is an aromatic nitrogen-, phosphorus- or sulphur-, preferably nitrogen-, containing ring, for example pyridine.

10. A method according to claim 8 or 9 in which the zwitterionic monomer is 2-methacryloyloxyethylphosphorylcholine.

11. A method according to any preceding claim in which the hydrophobic block is formed by radical polymerisation of ethylenically unsaturated monomers.

12. A method according to claim 11 in which the monomers from which the hydrophobic block is formed have the general formula VII Y¹B¹QV VII
in which Y¹ is an ethylenically unsaturated group selected from H₂C=CR⁴⁰-CO-A⁸-, H₂C=CR¹⁴-C₆H₄-A⁹-, H₂C=CR¹⁴-CH₂A¹⁰, F¹⁶O-CO-CR¹⁴=CF¹⁴-CO-O, R¹⁴CH=CH-CO-O-, R¹⁴CH=C(COOR¹⁶)CH₂-CO-O, 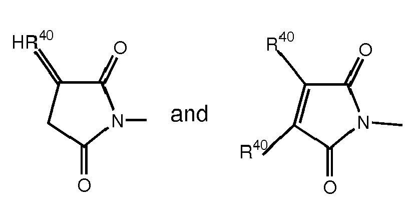
A⁸ is -O- or NR¹⁵;
A⁹ is selected from a bond, (CH₂)_{q}A¹⁰ and (CH₂)_{q} SO₃⁻ in which q is 1 to 12;
A¹⁰ is selected from a bond, -O-, O-CO-, CO-O-, CO-NR⁴¹-, -NR⁴¹-CO, O-CO-NR¹⁵-, NR¹⁵-CO-O-;
R¹⁴ is hydrogen or C₁₋₄alkyl;
R¹⁵ is hydrogen, C₁₋₄₋ alkyl or B¹Q;
R¹⁶ is hydrogen or C₁₋₄ alkyl;
B¹ is a bond, or a straight branched alkanediyl, alkylene oxaalkylene, or alkylene (oligooxalkylene) group, optionally containing one or more fluorine substituents; and
Q is a cationic or cationisable group of the formula -NR¹⁷ₚ, -PR¹⁷ₚ and SR¹⁷ᵣ, in which p is 2 or 3, r is 1 or 2, the groups R¹⁷ are the same or different and each is selected from the group consisting of hydrogen, C₁₋₂₄ alkyl and aryl, or two of the groups R¹⁷ together with the heteroatom to which they are attached from a 5 to 7 membered heterocyclic ring or three R¹⁷ groups together with the heteroatom to which they are attached form a 5 to 7 membered heteroaromatic ring, either of which rings may be fused to another 5 to 7 membered saturated or unsaturated ring, and any of the R¹⁷ groups may be substituted by amino or hydroxyl groups or halogen.

13. A method according any preceding claim wherein the hydrophobic block is formed from DPA or DEA monomers.

14. A method according to any preceding claim wherein after step (i), the cells are incubated for a period of time of at least 10 hours, preferably at least 24 hours.

15. A method according to any preceding claim, wherein the imaging agent is a fluorescent label, and the cells are imaged by monitoring the fluorescent emitted from the label.

16. A method according to any preceding claim wherein the imaging agent, once released into the cell, targets an organelle.

17. A method according to any preceding claim wherein the imaging agent is a biomolecule, or is derived from a biomolecule.

18. A composition comprising self-assembled constructs, and associated with the constructs, an imaging agent, wherein the constructs comprise an amphiphilic block copolymer having a hydrophilic and hydrophobic block,
and wherein the imaging agent is a biomolecule, or derived from a biomolecule, and is capable of targeting a specific cellular structure.

19. Composition according to claim 18, wherein the imaging agent is a fluorescent-labelled phospholipid, sphyngolipid, or cholesterol molecule.

20. Composition according to claim 18 or 19, wherein the amphiphilic block copolymer is as defined in any of claims 3-13.

21. A method for forming a composition as defined in any of claims 18-20 comprising the steps:
(i) dissolving the amphiphilic copolymer in an aqueous medium;
(ii) acidifying the composition formed in step (i);
(iii) adding the imaging agent to the composition; and
(iv) raising the pH to around neutral.

22. A method according to claim 21 comprising a preliminary step, before step (i), wherein the amphiphilic copolymer is dissolved in an organic solvent in a reaction vessel and the solvent is then evaporated to form a film on the inside of the reaction vessel.
